(19)

**Europäisches Patentamt**
**European Patent Office**
**Office européen des brevets**

(11) **EP 4 670 746 A1**

(12) **EUROPEAN PATENT APPLICATION**

(43) Date of publication:
**31.12.2025 Bulletin 2026/01**

(21) Application number: **24382694.8**

(22) Date of filing: **27.06.2024**

(51) International Patent Classification (IPC):
*A61L 9/04* (2006.01)    *A61L 9/012* (2006.01)
*A61K 8/04* (2006.01)    *A61Q 13/00* (2006.01)
*C11D 3/37* (2006.01)    *C11D 3/50* (2006.01)

(52) Cooperative Patent Classification (CPC):
**A61L 9/042; A61K 8/042; A61L 9/012; A61L 9/048;
A61Q 13/00; C11D 3/502; C11D 3/505;
A61L 2209/22**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **The Procter & Gamble Company
Cincinnati, OH 45202 (US)**

(72) Inventors:
• **FERNANDEZ PRIETO, Susana**
  **1853 Strombeek-Bever (BE)**
• **SMETS, Johan**
  **1853 Strombeek-Bever (BE)**
• **PIRONE, Domenico**
  **1853 Strombeek-Bever (BE)**
• **MARTINEZ HERNANDEZ, Jose Blas**
  **1853 Strombeek-Bever (BE)**
• **GUIMET, Isabelle**
  **1853 Strombeek-Bever (BE)**
• **BRENT, JR., John Leslie**
  **Cincinnati, 45202 (US)**
• **CORTES TRIVIÑO, Esperanza**
  **21071 Huelva (ES)**
• **MARTINEZ GARCIA, Inmaculada**
  **21071 Huelva (ES)**

(74) Representative: **P&G Patent Belgium UK
Temselaan 100
1853 Strombeek-Bever (BE)**

(54) **METHOD OF LOADING A VOLATILE HYDROPHOBIC MATERIAL INTO A NON-POROUS SOLID ARTICLE**

(57)    Provided herein is a method of preparing a product for the release of volatile hydrophobic material, comprising the steps:
i) providing a solid article comprising a polymer gel matrix formed from a chemically cross-linked material; and
ii) absorbing the volatile hydrophobic material into the solid article by contacting the solid article with liquid phase volatile hydrophobic material until the solid article comprises at least 5 wt. % of the volatile hydrophobic material, based on a final weight of the solid article, wherein:
the solid article is non-porous; and
the product is able to provide release of the volatile hydrophobic material by volatilization of the volatile hydrophobic material from the solid article at a temperature of 25°C, a relative humidity of 60%, and atmospheric pressure.

EP 4 670 746 A1

**Description**

FIELD OF THE INVENTION

The present invention relates to a method of preparing products for the release of volatile hydrophobic material. Suitable volatile hydrophobic material includes perfumes, insect repellents, essential oils, functional perfume components (FPCs), aesthetic agents, bioactive agents, malodor counteractants, and mixtures thereof.

BACKGROUND OF THE INVENTION

It is generally known to use a device to evaporate a volatile material into a space, particularly a domestic space, in order to deliver a variety of benefits, such as air freshening or perfuming of the air. Non-energized systems, for example, systems that are not powered by electrical energy, are a popular way for the delivery of volatile material into the atmosphere.

These systems can be classified into those that require human actuation, such as aerosols, and those which do not require human actuation, such as wick-based systems and gels. The first type delivers the volatile material on demand and the second type in a more continuous manner.

Variations on the second type of systems include polymer gel-based systems in which a volatile material is held within the matrix of a polymer gel (e.g. a polyurethane, polyester, or polyester-polyether). The volatile material may slowly be released from the polymer gel, providing sustained release over a desirable time period. Such products comprising a polyurethane polymer matrix are disclosed in US11173224B2. The volatile material that can be found in these products are incorporated during a polymer gel formulation step, which results in disadvantages. First, the curing of a polymer gel requires elevated temperatures that may cause degradation, premature loss by volatilisation, or a reduction in efficacy of the volatile hydrophobic material. Second, the presence of the volatile hydrophobic material and any additional solvent may hinder the gellification process. There is a need for improved methods that overcome some or all of the problems associated with the prior art.

SUMMARY OF THE INVENTION

It has surprisingly been found that loading a volatile hydrophobic material into a polymeric gel after a polymer curing step has been performed solves the problems discussed above. Since the volatile material is not present during the polymer gellification step, the volatile material (which may have solvent-like properties) does not interfere with the gellification process. As a result, the invention advantageously facilitates the preparation of polymer gel products from a wider range of starting materials than may be used with prior art methods. Loading a volatile hydrophobic material after a polymer curing step also prevents excessive loss or degradation of the volatile hydrophobic material during the curing step. The resulting volatile material-loaded polymeric gel products provide excellent release of the volatile hydrophobic material.

Thus, the present invention provides the following.

1. A method of preparing a product for the release of volatile hydrophobic material, comprising the steps:

i) providing a solid article comprising a polymer gel matrix formed from a chemically cross-linked material; and
ii) absorbing the volatile hydrophobic material into the solid article by contacting the solid article with liquid phase volatile hydrophobic material until the solid article comprises at least 5 wt. % of the volatile hydrophobic material, based on a final weight of the solid article,

wherein:

the solid article is non-porous; and
the product is able to provide release of the volatile hydrophobic material by volatilization of the volatile hydrophobic material from the solid article at a temperature of 25°C, a relative humidity of 60%, and atmospheric pressure.

2. The method according to clause 1, wherein contacting the solid article with liquid phase volatile hydrophobic material is conducted until the solid article comprises at least 15 wt. % of the volatile hydrophobic material, based on a final weight of the solid article.

3. The method according to clause 1 or 2, wherein absorbing the volatile hydrophobic material into the solid article comprises contacting at least 70% of a surface area of the solid article with the liquid phase volatile hydrophobic material,

optionally wherein absorbing the volatile hydrophobic material into the solid article comprises contacting at least 90% of a surface area of the solid article with the liquid phase volatile hydrophobic material.

4. The method according to any one of the preceding clauses, wherein the solid article is contacted with a liquid phase volatile hydrophobic material that comprises no more than 10 wt. % solvent,

   optionally no more than 5 wt. % solvent;
   more optionally no more than 3 wt. % solvent,
   most optionally no more than 1 wt. % solvent.

5. The method according to any one of the preceding clauses, wherein step ii) comprises immersing the solid article in the volatile hydrophobic material,

optionally wherein the method further comprises the steps:

   iiia) removing the solid article from the volatile hydrophobic material; and
   iva) drying the solid articles to remove surface liquid volatile hydrophobic material.

6. The method according to clause 5, wherein step ii) is conducted at a temperature of from 10°C to 70°C,

   optionally at a temperature of from 15°C to 60°C,
   more optionally at a temperature of from 20°C to 55°C.

7. The method according to clause 5 or 6, wherein step ii) is conducted:

   until the solid article comprises at least 25 wt. % (e.g. at least 30 wt. %) of the volatile hydrophobic material, based on a final weight of the solid article; and/or
   for a time period of from 30 minutes to 24 hours.

8. The method according to any one of clauses 1 to 4, wherein step ii) comprises allowing the volatile hydrophobic material to be absorbed by the solid article under a reduced pressure.

9. The method according to clause 8, wherein step ii) comprises:

   iia) placing the solid article and volatile hydrophobic material into a vacuum bag;
   iib) applying the reduced pressure to the vacuum bag and sealing the vacuum bag;
   iic) allowing the volatile hydrophobic material to be absorbed into the solid article until the solid article comprises at least 5 wt. % of the volatile hydrophobic material, based on a final weight of the solid article.

10. The method according to clause 8 or 9, wherein the reduced pressure is a pressure of from $10^{-8}$ mbar ($10^{-9}$ kPa) to about 10 mbar (1 kPa).

11. The method according to any one of clauses 8 to 10, wherein the step of allowing the volatile hydrophobic material to be absorbed by the solid article under a reduced pressure is conducted at a temperature of from 30°C to 80°C,

   optionally from 40°C to 70°C,
   more optionally from 45°C to 60°C,
   such as about 50°C.

12. The method according to any one of clauses 8 to 11, wherein the step of allowing the volatile hydrophobic material to be absorbed by the solid article under a reduced pressure is conducted:
until the solid article comprises at least 20 wt. % (e.g. at least 25 wt. %) of the volatile hydrophobic material, based on a final weight of the solid article; and/or
for a time period of from 60 minutes to 600 minutes.

13. The method according to any one of the previous clauses, wherein the volatile hydrophobic material does not include pharmaceutically effective amounts of pharmaceutically active compounds.

14. The method according to any one of the previous clauses, wherein the chemically cross-linked material is derived from a natural oil,
optionally wherein the chemically cross-linked material is selected from the group consisting of a polyurethane, a polyester, and a polyester-polyether copolymeric material.

15. The method according to any one of the previous clauses, further comprising a step of heating the solid article to a temperature of at least 70°C before contacting the solid article with the volatile hydrophobic material.

16. The method according to clause 15, wherein the solid article is heated to a temperature of from 80°C to 120°C for a time period of from 3 minutes to 30 minutes.

17. A solid article obtainable by a method according to any one of the preceding clauses, wherein the solid article comprises at least 5 wt. % of the volatile hydrophobic material, based on a final weight of the solid article.

18. Use of a solid article according to clause 17 for the release of a volatile hydrophobic material,
optionally wherein the volatile hydrophobic material is selected from the group consisting of a perfume, an essential oil, an insect repellant and a pheromone.

DETAILED DESCRIPTION OF THE INVENTION

The invention provides a method of preparing a product for the release of volatile hydrophobic material, comprising the steps:

i) providing a solid article comprising a polymer gel matrix formed from a chemically cross-linked material; and
ii) absorbing the volatile hydrophobic material into the solid article by contacting the solid article with liquid phase volatile hydrophobic material until the solid article comprises at least 5 wt. % of the volatile hydrophobic material, based on a final weight of the solid article,

wherein:

the solid article is non-porous; and
the product is able to provide release of the volatile hydrophobic material by volatilization of the volatile hydrophobic material from the solid article at a temperature of 25°C, a relative humidity of 60%, and atmospheric pressure.

Thus, the invention provides a method of preparing a product that is suitable for the release of volatile hydrophobic material to a surrounding environment. The product is prepared by absorbing volatile hydrophobic material into a solid article. The step of absorbing the volatile hydrophobic material into the solid article may be referred to herein as "loading" the solid article with volatile hydrophobic material.
The solid article described herein is non-porous, and may be referred to herein as a "non-porous solid article". However, for the sake of brevity, the non-porous solid article may also be referred to herein simply as the "solid article". As used herein, "non-porous" means that the solid article does not comprise pores or connected micro-channels distributed throughout substantially all of (e.g. all of) the solid article. Thus, the solid article may have a porosity of less than 15%, preferably less than 10%, such as less than 5%, such as less than 3%, such as less than 1% based on total sample weight as described in method ASTM D4404-18 for Determination of Pore Volume and Pore Volume Distribution of Soil and Rock by Mercury Intrusion Porosimetry.
The product for the release of volatile hydrophobic material is for releasing the volatile hydrophobic material to a surrounding atmosphere. Thus, the product is able to provide release of the volatile hydrophobic material by volatilization of the volatile hydrophobic material from the solid article at a temperature of 25°C, a relative humidity of 60%, and atmospheric pressure. In some configurations, the product may be an air-freshening product that is able to release an air-freshening composition to a surrounding environment. In some configurations, the product may be an insect-behavior modifying product (such as an insect-repelling product, or an insect-attracting product), that is able to release a material that modifies the behavior of insects to a surrounding environment. Such products include insect-repelling products (e.g. for repelling insects away from an area), and insect-attracting products (e.g. for use in insect traps).
In some configurations, the product may provide sustained release of the volatile hydrophobic material over a time period of at least two weeks. In some configurations, the product may provide sustained release of the volatile hydrophobic material over a time period of at least four weeks. In some configurations, the product may provide sustained release of the volatile hydrophobic material over a time period of at least six weeks. In some configurations, the product may provide sustained release of the volatile hydrophobic material over a time period of at least eight weeks. Sustained release may refer to the release of the volatile hydrophobic material over a specified time period, where the effects provided by the volatile hydrophobic material (e.g. air freshening or insect repelling) are obtained for the entire time period in question. Therefore, in some configurations, sustained release may refer to release where the volatile hydrophobic composition is not depleted before the end of the time period in question. In some configurations, sustained release may refer to release of at least 50%, but not more than 99% of the volatile hydrophobic material, during the time period in question. In some configurations, sustained release may refer to release of at least 60%, but not more than 95% of the volatile hydrophobic material during the time period in question.

The method of the invention may be tuned to provide a product that is able to release a desired amount of a hydrophobic material over a specific time period. The release rate of the hydrophobic material may be controlled by changing the conditions of the method of the invention, and/or by changing the identity of the hydrophobic material and/or the chemical structure or size of the solid article. In particular, this may involve changing the nature of interactions between the hydrophobic material and the gel matrix (e.g. by changing the hydrophobicity/hydrophilicity of the hydrophobic material and/or gel matrix), as well as the mesh size of the gel matrix (e.g. by controlling the degree of crosslinking). Thus, the invention may be used to provide products that will release a hydrophobic material very quickly (e.g. within a period of a one to two weeks), or over a longer time period (e.g. at least eight weeks or at least twelve weeks).

Any volatile hydrophobic material that may be desirable to release to a surrounding environment may be used in the method of the invention. Thus, the volatile hydrophobic material may include perfumes, essential oils, functional perfume components (FPCs), aesthetic agents, bioactive agents, malodor counteractants, insect repellents, pheromones, and mixtures thereof. Particularly suitable components of the volatile hydrophobic material include perfumes, essential oils, malodor counteractants, and pheromones.

The volatile hydrophobic material may have a boiling point of less than 450 °C, preferably from 60 °C to 400 °C, more preferably from 75 °C to 380 °C, where all boiling points are determined at atmospheric pressure. In addition, or alternatively, the volatile hydrophobic material may have a vapor pressure of at least $10^{-6}$ Torr at 25°C, such as at least $10^{-5}$ Torr at 25°C, or at least $10^{-4}$ Torr at 25°C.

Nevertheless, the volatile hydrophobic material may comprise components that are non-volatile or have low volatility (e.g. a boiling point of over 350 °C). Such non-volatile or low volatility components can be as eluents for perfumes and perfume mixtures.

As mentioned, the volatile hydrophobic material may comprise a pheromone. As used herein, a "pheromone" refers to a non-human pheromone. Exemplary use cases for pheromones include repelling insects from crops, attracting insects to traps, changing the behaviour of household pets (e.g. training and/or pacifying household pets), and changing the behaviour of birds (e.g. attracting or repelling birds from a garden).

Exemplary environments into which it may be desirable to release volatile hydrophobic material include interior spaces (e.g. rooms and cars); bags (e.g. sports bags, purses, backpacks and luggage); damp household environments (e.g. bathroom areas, washing machines, dishwashers, sinks and drains); interiors of household furniture (e.g. drawers, closets, cabinets); trash cans and bins; pet relates items (e.g. dog baskets and cat litterboxes); outside areas (e.g. gardens, patios, and crop fields).

The volatile hydrophobic material preferably does not include pharmaceutically effective amounts of pharmaceutically active compounds. Thus, the product for the release of volatile hydrophobic material is preferably not a pharmaceutical product, e.g. is not a transdermal patch. While the volatile hydrophobic material may include compounds that are known to have a pharmaceutical effect, such as menthol, use of the product obtained by the method of the invention will not provide such compounds to humans in pharmaceutically effective amounts. For the avoidance of doubt, as used herein, a non-human pheromone is not a "pharmaceutically active compound" or a "pharmaceutical product".

The method of the invention includes providing a solid article comprising a polymer gel matrix formed from a chemically cross-linked material. As defined herein, a "solid article" is a product that is solid, i.e. the product does not flow and maintains its shape when stored at 25°C. The solid article is typically a self-supporting solid. For the avoidance of doubt, the solid article may be flexible and/or viscoelastic.

The polymer gel matrix may comprise any appropriate polymeric chains and crosslinks, and nonlimiting examples include polyester materials, polyester-polyether copolymeric materials, and polyurethane materials. Nevertheless, a skilled person will appreciate that solid articles comprising other polymer matrices may be used in the method of the invention.

In some configurations, the chemically cross-linked material may be derived from a natural oil (e.g. a vegetable oil). The natural oil may be any natural oil disclosed herein. The natural oil may be chemically modified before preparation of the polymer gel matrix, e.g. a natural oil may be an epoxidized natural oil, which may be obtained by methods known to a person skilled in the art.

Natural oils such as vegetable oils may be used to prepare polyurethane, polyester and polyester-polyether chemically cross-linked materials, such as by the methods disclosed herein and in US11173224B2.

A polyurethane may be obtained by cross-linking a polyol vegetable oil (e.g. castor oil) with a diisocyanate. Castor oil is particularly suitable for preparing polyurethanes, though any polyol vegetable oil may be used. Castor oil (ricinus oil) is a pale yellow and viscous liquid, derived from the bean of the castor plant (ricinus communis). Castor oil is predominately made up of triglycerides of fatty acids that contain 87-90% of ricinoleic acid (cis-12-hydroxyoctadec-9-enoic acid) and can be achieved in high purity grades. Castor oil and its derivatives have been used as polyols for polyurethanes and adhesives. The castor oil can be partially hydrogenated. It has been found that castor oil provides the length of the branches and the position of the hydroxyl groups which is particularly suited for providing a chemically cross-linked gel having a pore size which results in slow release of the hydrophobic material, particularly where the hydrophobic material is a perfume. In addition, the chemically cross-linked gels derived from castor oil show less syneresis of the volatile hydrophobic material from the gel.

A polyester material may be obtained by cross-linking an epoxidized vegetable oil with a polyfunctional carboxylic acid. Suitable epoxidized vegetable oils include linseed oil, epoxidized soybean oil, epoxidized castor oil, epoxidized rapeseed oil, epoxidized vernonia oil, vernonia oil, epoxidized corn oil, epoxidized cottonseed oil, epoxidized canola oil, epoxidized grape seed oil, epoxidized poppy seed oil, epoxidized tung oil, epoxidized sunflower oil, epoxidized safflower oil, epoxidized walnut oil, and any combinations thereof. Suitable polyfunctional carboxylic acids include citric acid, maleic acid, malonic acid, itaconic acid, fumaric acid, succinic acid, methyl succinic acid, malic acid, phthalic acid, tartaric acid, citraconic acid, furan dicarboxylic acid, and any combinations thereof.

A polyester-polyether material may be obtained by:

(i) reacting a starting material with an acid anhydride to form an intermediate, and
(ii) cross-linking the intermediate with a polyepoxide;

wherein said starting material comprises at least 2 (e.g. from 2 to 12, such as from 2 to 8, or from 2 to 6) functional groups selected from the group consisting of hydroxyl, alkene, conjugated diene, alkyne and combinations thereof.

Suitable starting materials for preparing a polyester-polyether material include castor oil, sunflower oil, tung oil, vernonia oil, linseed oil, polyethylene glycol, linoleic acid, arachidonic acid, eicosapentaenoic acid, ricinoleic acid, soybean oil, palm oil, olive oil, corn oil, canola oil, rapeseed oil, coconut oil, cottonseed oil, palm kernel oil, rice bran oil, safflower oil, sesame oil, tall oil, lard, tallow, fish oil, oils from algae, pentaerythritol, sorbitol, malitol, sucrose, glucose, trehalose, galactose, and combinations thereof. Suitable acid anhydrides include maleic anhydride, succinic anhydride, phthalic anhydride, hexahydrophthalic anhydride, trimellitic anhydride chloride, methyltetrahydrophthalic anhydride, acrylic anhydride, itaconic anhydride, dodecenylsuccinic anhydride, 1,2,4-eenzenetricarboxylic anhydride, 2,3-dimethylmaleic anhydride, phenyl succinic anhydride, 1,2,3,6-tetrahydrophthalic anhydride, bromomaleic anhydride, diglycolic anhydride, and any combinations thereof. Suitable polyepoxides include polyethylene glycol diglycidyl ether (PEGDGE), propylene glycol diglycidyl ether (PPGDGE), butanediol diglycidyl ether, neopentyl glycol diglycidyl ether, trimethylolpropane triglycidyl ether, resorcinol diglycidyl ether, and any combinations thereof.

In some configurations, the solid article is not a silicone material (e.g. does not comprise silicone). In some configurations, the solid article is not a polyacrylate or polymethacrylate material (e.g. does not comprise polyacrylate or polymethacrylate). Without being bound by theory, silicones and polyacrylates/polymethacrylates are believed to provide inferior absorption of volatile hydrophobic material as compared to the solid articles disclosed herein.

The solid article may be a solid polymeric material, such as a solid polymeric material comprising a polymeric system with a correlation length as measured by Small-angle X-ray scattering (SAXS) of less than or equal to 20 nm, such as from about 0.05 nm to 16 nm, preferably from 0.08 nm to 10 nm, even more preferably from about 0.1 to 4 nm.

The solid article may have any appropriate shape, including but not limited to: discs, hemispheres, beads (whether spherical, spheroid or ovoid), and polygonal shapes (whether regular or irregular. The solid article may have any suitable size. Since the solid articles release the hydrophobic material from the surface of the solid article (e.g. by evaporation), the surface area of the solid article will affect the release rate of the hydrophobic material. For instance, for the same mass of the solid article, thin sheets result in faster hydrophobic material release and therefore a quicker depletion of volatile material than thicker shapes such as cuboids or spheres. For typical applications, the solid article may have a surface area of less than about 1250 $cm^2$, such as from about 0.2 $cm^2$ to about 150 $cm^2$, e.g. from about 0.5 $cm^2$ to about 100 $cm^2$. For typical applications, the solid article may have a volume of from about 7 $mm^3$ to about 300 $cm^3$, such as about 0.02 $cm^3$ to about 150 $cm^3$. Nevertheless a person skilled in the art will appreciate that solid articles of other sizes may be useful for certain applications.

As discussed herein, products obtained by the method of the invention may be used to release the volatile material to a surrounding environment. The time period for which such a product is able to release volatile material may be referred to as the product's "lifetime". The lifetime of a product obtained by the method of the invention will be influenced by the release rate of hydrophobic material, and the total amount of hydrophobic material present. Since the release rate will depend on the surface area, and the total amount of hydrophobic material present may depend on the volume of the product (for any given concentration of hydrophobic material), this principle may be understood by reference to surface area:volume ratio. In order to maintain a desirable balance between product lifetime, release rate and overall dimensions, for typical applications it may be desirable for the solid article to have a surface area:volume ratio of from about 2 $cm^{-1}$ to

about 30 cm$^{-1}$, such as about 4 cm$^{-1}$ to about 25 cm$^{-1}$. Nevertheless, a skilled person will appreciate that the solid articles having other surface area:volume ratios may be useful in certain applications.

As will be appreciated by a person skilled in the art, one or more solid articles may be used to provide a desired release of hydrophobic material in an environment. For example, if a very high release rate is desired then one may use multiple solid articles each having a relatively low volume, but with a very high surface area for said volume (i.e. a high surface area:volume ratio), as compared to using a single solid article. In these cases, the lifetime of the solid articles may be lower, on account of a higher evaporation rate resulting from the increased surface area. A skilled person will appreciate that the opposite situation may also apply, and a solid article having a low surface area:volume ratio may be used in a situation where a lower release rate is desired.

The liquid phase volatile hydrophobic material may be a mixture of volatile hydrophobic compounds, and may include compounds that are solid at standard temperature and pressure (e.g. menthol). In such cases, the presence of volatile hydrophobic compounds that are liquid at standard temperature and pressure will typically solubilise any solid volatile hydrophobic compounds, avoiding the need for a solvent. In this context, "solvent" may be understood to mean a material that is included in the mixture for the primary purpose of solubilising other components of the mixture, and which does not provide other benefits when released into a surrounding environment, such as air freshening, insect attracting or repelling, or acting as a pheromone.

Thus, the methods disclosed herein may be conducted in the presence of low levels of solvent, or in the absence of a solvent. For example, the solid article may be contacted with liquid phase volatile hydrophobic material that comprises no more than 10 wt. % solvent (such as no more than 5 wt. % solvent, no more than 3 wt. % solvent, no more than 1 wt. % solvent, or in the absence of solvent). This ensures that the absorbent capacity of the solid article is used to absorb volatile hydrophobic material, that may be subsequently desirably released to perform a function (e.g. freshening the air or releasing an insect repellent or pheromone), and the solid article does not become saturated with a solvent that provides minimal functional benefit. Nevertheless, a solvent may be used in certain circumstances in order to improve the absorption of volatile hydrophobic material.

The method of the invention includes a step of absorbing the volatile hydrophobic material into the solid article by contacting the solid article with liquid phase volatile hydrophobic material until the solid article comprises at least 5 wt. % of the volatile hydrophobic material, such as at least 10 wt. %, at least 15 wt. %, at least 20 wt. %, at least 25 wt. %, at least 30 wt. %, at least 40 wt. %, at least 45 wt. %, at least 50 wt. %, at least 55 wt. %, at least 60 wt. %, at least 65 wt. %, at least 70 wt. %, at least 75 wt. %, or at least 80 wt. %, based on the final weight of the solid article loaded with volatile hydrophobic material. In general, a higher loading of volatile hydrophobic material may be desirable to improve performance in releasing volatile hydrophobic material to an environment, such as freshening a space or releasing a pheromone to a crop field. In some configurations, step ii) may be conducted until the solid article comprises at least 40 wt. % of volatile hydrophobic material. In some configurations, step ii) may be conducted until the solid article comprises at least 50 wt. % of volatile hydrophobic material. In some configurations, step ii) may be conducted until the solid article comprises at least 60 wt. % of volatile hydrophobic material. In some configurations, step ii) may be conducted until the solid article comprises at least 70 wt. % of volatile hydrophobic material. While an increased loading of volatile hydrophobic material is correlated with increased product lifetime, from a perspective of balancing manufacturing time and cost with product efficacy, there may be circumstances where a non-maximal loading of volatile hydrophobic material is desirable.

For the avoidance of doubt, the end point of any range disclosed herein may be combined with any end point of any other range disclosed herein in respect of the same variable. Thus, based on the above, the method of the invention may involve contacting the solid article with liquid phase volatile hydrophobic material until the solid article comprises:

from 5 wt. % to 10 wt. %, from 5 wt. % to 15 wt. %, from 5 wt. % to 20 wt. %, from 5 wt. % to 25 wt. %, from 5 wt. % to 30 wt. %, from 5 wt. % to 40 wt. %, from 5 wt. % to 45 wt. %, from 5 wt. % to 50 wt. %, from 5 wt. % to 55 wt. %, from 5 wt. % to 60 wt. %, from 5 wt. % to 65 wt. %,
from 5 wt. % to 70 wt. %, from 5 wt. % to 75 wt. %, from 5 wt. % to 80 wt. %, of the volatile hydrophobic material based on the final weight of the solid article loaded with volatile hydrophobic material. Corresponding ranges with lower bounds of 10, 15, 20, 25, 30, 35, 40, 45, 50, 55, 60, 65, 70 and 75 wt. %, respectively, are also to be considered disclosed herein.

In order to prevent buckling or deformation of the solid article, it may be desirable for the volatile hydrophobic material to be absorbed into the solid article from substantially the entire surface area of the solid article. Thus, in some configurations, absorbing the volatile hydrophobic material into the solid article may comprise contacting at least 70% (such as at least 90%, at least 95%, or about 100%) of a surface area of the solid article with liquid phase volatile hydrophobic material.

In order to ensure effective contact between the solid article and the volatile hydrophobic material, the step of absorbing the volatile hydrophobic material into the solid article may comprise immersing the solid article in the volatile hydrophobic material. The immersion is preferably a total immersion. Thus, in some configurations the immersion comprises contacting the entire surface of the solid article with the liquid volatile hydrophobic material. When an immersion step is conducted, the method may comprise the further steps of:

iiia) removing the solid article from the volatile hydrophobic material; and

iva) drying the solid articles to remove surface liquid volatile hydrophobic material.

The drying step may be conducted by contacting the solid article with an absorbent/wicking product such as a paper towel, to physically remove the volatile hydrophobic material. Alternatively, the drying step may be conducted by exposing the solid article to an airflow or elevated temperature to evaporate/volatilise excess volatile hydrophobic material. It is desirable that the drying step is conducted only for as long as is required to dry the surface of solid article, and does not result in significant release or removal of volatile hydrophobic material from within the solid article.

The immersion may be conducted at any appropriate temperature, though a slightly elevated temperature may be preferable to increase the rate of absorption. Thus, the immersion may be conducted at a temperature of from 10°C to 70°C, such as at a temperature of from 15°C to 60°C, or at a temperature of from 20°C to 55°C. The temperature will typically be selected to be at least 15°C below the flash point of the liquid phase volatile hydrophobic material.

The immersion may be conducted until the solid article comprises a desired amount of hydrophobic material, such as at least 15 wt. %, at least 25 wt. %, at least 30 wt. %, e.g. from 25 wt. % to 85 wt. % (such as 40 wt. % to 75 wt. %) of the volatile hydrophobic material, based on the final weight of the solid article (i.e. the total weight of the solid article and the absorbed volatile hydrophobic material).

The immersion may be conducted for any appropriate time period, for example from 30 minutes to 24 hours, such as from 1 hour to 10 hours, or from 2 hours to 6 hours (e.g. about 4 hours).

Another method to ensure effective contact between liquid phase volatile hydrophobic material and the solid article is placing the solid article and liquid phase volatile hydrophobic material into a vacuum bag, and removing the air by applying a reduced pressure. After application of a vacuum and subsequently sealing the vacuum bag, the vacuum bag will have compressed to closely conform to the shape of the solid article. This ensures that the liquid phase volatile hydrophobic material will be forced over the surface of the solid article, enabling a high degree of contact (e.g. contact with about 100% of the surface area of the solid article). This method allows a very high degree of contact between the solid article and liquid phase volatile hydrophobic material, whilst using a lower amount of liquid phase volatile hydrophobic material than would be required for total immersion. In addition, the mechanical pressure of the vacuum bag pressing against the solid article may help to force the liquid phase volatile hydrophobic material into the solid article, facilitating absorption.

Therefore, in some configurations, step ii) may comprise:

iia) placing the solid article and volatile hydrophobic material into a vacuum bag;

iib) applying a reduced pressure to the vacuum bag;

iic) allowing the volatile hydrophobic material to be absorbed into the solid article until the solid article comprises at least 5 wt. % of the volatile hydrophobic material, based on the final weight of the solid article.

For brevity, the method comprising steps iia) to iic) may be referred to herein as the "vacuum method".

Any appropriate vacuum bag may be used in the method of the invention, such as those available from Parkside (Malaysia), Bolsemack (Spain) or Stroebel (Germany). Suitable vacuum bags may be made from any appropriate material, such as polyamide, polyethylene, polypropylene, cellulose and mixtures thereof. In some configurations, the vacuum bag may have appropriate barrier properties, such as any or all of the following.

- A mineral oil saturated hydrocarbon (MOSH) migration according to DIN14338 (Tenax method) of less than 2.0 mg/kg.
- A mineral oil aromatic hydrocarbon (MOAH) migration according to DIN14338 (Tenax method) of less than 0.5 mg/kg.
- An air permeability (TAPPI T 547 Om-2012 (R2018)) of less than 0.1 ml/min.

Any reduced pressure that ensures that the vacuum bag closely conforms to the shape of the solid article may be used. Exemplary reduced pressures include those in the range of from about $10^{-8}$ mbar to about 10 mbar, from about $10^{-7}$ mbar to about 0.1 mbar, from about $10^{-6}$ mbar to about $10^{-3}$ mbar, but a skilled person will appreciate that the effects of the invention may be obtained using pressures outside this range.

Absorption of volatile hydrophobic material in the vacuum method may preferably be conducted at an elevated temperature, in order to increase the rate of absorption. Thus, the absorption step iic) may be conducted at a temperature of from 30°C to 80°C, such as from 40°C to 70°C, such as from 45°C to 60°C, e.g. about 50°C.

Absorption of the volatile hydrophobic material in the vacuum method may be conducted until the solid article comprises a desired amount of hydrophobic material, such as at least 15 wt. %, or at least 25 wt. %, for example from 15 wt. % to 75 wt. % (e.g. 25 to 60 wt. %) of the volatile hydrophobic material, based on the final weight of the solid article (i.e. the total weight of the solid article and the absorbed volatile hydrophobic material). The absorption may be conducted for any appropriate time period, such as from 60 minutes to 600 minutes. In some configurations, an appropriate loading of volatile hydrophobic material may be achieved after around 120 minutes.

The vacuum method may further provide the advantage that the vacuum bag may also function as appropriate packaging for the product to be sold. Thus, the step of absorbing the volatile hydrophobic material into the solid article may also serve as a packaging step for the final product. This removes the need for a separate packaging step, improving the efficiency of the production process.

During the absorption of volatile hydrophobic material into the solid article, the volatile hydrophobic material will become embedded in the polymer gel matrix. The rate of absorption may therefore be influenced by the packing of the polymer chains within the gel matrix. In order to increase the ease of absorbing volatile hydrophobic material, the solid article may be pre-treated by heating to a temperature that causes expansion of the polymer gel matrix, increasing the distance between polymer chains and facilitating absorption of volatile hydrophobic material. Thus, the method of the invention may comprise a step of heating the solid article to a temperature of at least 70°C before contacting the solid article with the volatile hydrophobic material. The heating step may be conducted for any appropriate time, such as at least 1 minute, at least 3 minutes, at least 5 minutes. Typically, the heating step will be conducted for a time that is no longer than 30 minutes (such as a time period of from 3 minutes to 30 minutes), though longer times may be appropriate in some circumstances. As will be appreciated by a person skilled in the art, the required heating time may depend on the size of the solid article. The heating step may be conducted at any appropriate temperature, such as from 80°C to 120°C, e.g. about 100°C.

The absorption of volatile hydrophobic material into the solid article may be conducted at a pressure of about atmospheric pressure, or below atmospheric pressure. Typically, the absorption step is not conducted at a pressure that is substantially greater than atmospheric pressure (e.g. 10% above atmospheric pressure or greater). Typically, the absorption step is not conducted while any material is maintained at or near supercritical conditions. When the absorption step is conducted by immersion as described herein, the immersion is typically conducted at atmospheric pressure.

The invention is illustrated in more detail with reference to the below Examples, which are not to be construed as limitative.

EXAMPLES

CHARACTERISATION METHODS

**General Method A**: **Determining the microstructure/correlation length (via SAXS)**

Sample preparation: A small (~1mm x~1mm x ~3mm) segment of gel is cut using a scalpel and it is placed into a demountable cell with Kapton film windows giving a sample thickness of 1 mm. SAXS measurements are performed using a HECUS, S3-MICRO Kratky-type camera equipped with a position sensitive, 50M OED detector comprising of 1024 channels, 54 $\mu$m in width. An ultra-brilliant point microfocus X-ray source (GENIX-Fox 3D, Xenocs, Grenoble) provides Cu K$\alpha$ radiation with a wavelength, $\lambda$, of 1.542 Å at a maximum power of 50 W. A sample-to-detector distance of 281 mm allows for a measurable q-range between 0.01 and 0.54 Å-1 (where q, the scattering vector, is given by q = 4$\pi$/$\lambda$ sin $\theta$, and 2$\theta$ is the scattering angle). The S3-MICRO camera is calibrated using silver behenate (d = 58.38 Å) and kept under vacuum to reduce scattering from air. Measurements are performed at a temperature of 25 °C and controlled by a Peltier element with an accuracy of 0.1 °C. Raw scattering data is corrected for the scattering of the cell and unreacted pure castor oil using a relative transmission factor.

Data analysis: The intensity of scattered radiation, I(Q), for a typical gel has a Q-dependence following a Lorentzian form (equation 1.0) (Hammouda, B. Insight into Clustering in Poly(ethylene oxide) Solutions. Macromolecules 2004. 37, 6932-6937):

$$I(Q) = \frac{I(0)}{1+(Q\xi)^2}$$

wherein,

I (Q) is intensity of scattered irradiation
I (0) is the scattering length intensity at q=0
$\xi$ is the correlation length

This describes scattering from a polymer where the polymer chains are considered as a "blob" of size $\xi$. A plot of $1/I(Q)$ vs. $Q2$ will yield a straight line in the low q region with a intercept of $1/I(0)$ and slope of $\xi2/I(0)$ from which the correlation length can be obtained.

PREPARATION METHODS

**General Method B: Synthesis of solid articles**

The solid articles in the below Examples were prepared by the following steps:

(i) mixing raw materials except the volatile hydrophobic material to form a partially polymerized intermediate mixture,
(ii) placing the partially polymerized intermediate mixture into one or more molds (e.g. having a desired shape), and

iii) curing the partially polymerized intermediate at elevated temperature.

The nature of step (i) will depend on the specific raw materials used.

- When the solid article comprises a polyester matrix, step (i) may comprise mixing an epoxidized vegetable oil (e.g. epoxidized soybean oil) with a polyfunctional carboxylic acid (e.g. citric acid) in an appropriate solvent (e.g. water).
- When the solid article comprises a polyester-polyether matrix, step (i) may comprise:

(ia) reacting a starting material (e.g. castor oil) with an acid anhydride (e.g. maleic anhydride) to form an intermediate compound, where the starting material comprises at least 2 functional groups selected from the group consisting of hydroxyl, alkene, conjugated diene, alkyne and combinations thereof; and
(ib) reacting the intermediate compound with a polyepoxide comprising at least two epoxide rings (e.g. polyethylene glycol diglycidyl ether).

- When the solid article comprises a polyurethane matrix, step (i) may comprise mixing a polyol (e.g. castor oil) with a diisocyanate.

Step (i) may typically be performed at elevated temperature, such as a temperature of from 60 to 150°C, preferably from 80 to 120°C. Step (i) may typically be performed until the reaction mixture has reached a specific viscosity that indicates partial polymerization, such as a viscosity of from 30 to 80 cP (such as 40 to 60 cP) at the reaction temperature.

Step (ii) may typically be performed at a lower temperature than step (i), so as to slow down the polymerization reaction whilst filling in the molds (e.g. a silicon mold) of a desired size and shape. Thus, step (ii) may be performed at a temperature of from 20 to 80°C, preferably from 30 to 50°C.

Step (iii) represents a curing step, and may be performed at any appropriate temperature and for any appropriate duration so as to allow for appropriate curing of the mixture to provide a solid article. Thus, step (iii) may be performed at a temperature of from 60 to 120°C for a period of from 4 to 48 hours, such as from 12 to 36 hours, or from 18 to 30 hours.

**General Method C: Total Immersion Method for Loading Volatile Hydrophobic Material**

Unloaded solid articles are placed in a suitable vessel (e.g. glass) with a cap/lid, where the diameter of the vessel is at least twice as large as the largest horizontal dimension of the solid article. The liquid volatile hydrophobic material is added to a level that is at least 3 mm higher than the highest dimension of the solid article. Absorption is carried out between 20°C and 55°C for a set time period (e.g. until a desired degree of loading has been reached). The solid articles loaded with volatile hydrophobic material are removed from the glass vessel and excess volatile hydrophobic material is gently removed using a cellulose adsorbent soft paper towel.

The loading percentage is determined by weighing the loaded solid articles and calculated using following formula:

$$\% \, wt \, loading = \frac{Wf - Wi}{Wf} \, x100$$

wherein,

Wf is the final weight in grams of the solid article after loading with the volatile material; and

Wi is the initial weight in grams of the solid article before immersion in the volatile hydrophobic material.

The final loading percentage will be affected by the identity of the volatile hydrophobic material, the absorption temperature and the immersion time. The precise timings and temperatures used may be varied based on the properties of the solid article and volatile hydrophobic material used.

### General Method D: Vacuum Method for Loading Volatile Hydrophobic Material

The solid article is placed into a vacuum bag and then the desired amount of volatile hydrophobic material is added into the bag in liquid form. For the avoidance of doubt, the volatile hydrophobic material may be a mixture, and the mixture may comprise components that are solid at standard temperature and pressure, provided that the final mixture is in liquid form. The bag comprising the solid article and the volatile hydrophobic material is placed in a vacuum sealer machine (e.g. Edenox Vaksicc - 20 2A E), and a reduced pressure of 1.3 kPa is applied for 90 seconds. The bag is then sealed over 5 seconds with an additional 2.5 seconds to allow the seal to cool to 20°C. Then, the sealed bags comprising the solid article and the volatile hydrophobic material at reduced pressure are placed in an oven at 55°C for 120 minutes.

The loading percentage is determined by weighing the loaded solid articles and calculated using following formula:

$$\% \, wt \, loading = \frac{Wf - Wi}{Wf} x 100$$

wherein,

Wf is the final weight in grams of the solid article after loading with the volatile material and when extracted from the bag; and
Wi is the initial weight in grams of the solid article before being placed into the bag.

The final loading percentage will be affected by the identity of the volatile hydrophobic material, the absorption temperature and the oven duration. The precise timings and temperatures used may be varied based on the properties of the solid article and volatile hydrophobic material used.

An optional pre-treatment step may be conducted before the vacuum loading. Thus, the solid articles may be preheated at 100°C for 5 minutes before being placed into the vacuum bags. The pre-heating step is believed to expand the polymeric matrix of the s solid articles, facilitating the absorption of volatile hydrophobic material.

TESTING METHODS

### General Method E: Determining volatile hydrophobic material release

The ability of a solid article to release volatile hydrophobic material was determined by weight loss measurements of the solid article after storage at $45 \pm 2$ °C, 60% relative humidity and atmospheric pressure for specified time period. The results were normalized to mg/h per gram of solid article (initial weight of the loaded article).

MATERIALS

Materials were obtained from the following suppliers.

Castor oil was obtained from Guinama
Soybean epoxidized oil (oxirane oxygen content of 6.6%) was obtained from Xiamen through CFT (China).
DESMODUR® eco N 7300 was obtained from Covestro (Germany)
Citric acid obtained from Sigma-Aldrich (USA).
Maleic anhydride oil was obtained from Sigma Aldrich
Polyethylene glycol diglycidyl ether (Mn 500 was obtained from Sigma Aldrich Tetra-n-butylammonium bromide, 98+% was obtained from Alfa Aesar
PPO: Peppermint oil, Ventos, Spain
SPO: Spearmint oil, Ventos, Spain
CNO: Cinnamon oil, Ventos, Spain
A: Proprietary perfume composition

B: Proprietary perfume composition (different to A)

EXAMPLES 1-12

The solid articles used in Examples 1 to 12 (Table 1) were prepared according to General Method B. They were loaded with volatile hydrophobic material according to General Method C (immersion method). In Examples 1 to 8, the solid article has the shape of a hemisphere with a diameter of 5 mm and a total surface area of 50.9 mm$^2$ before the immersion in the volatile hydrophobic material. In Examples 9 to 12, the solid article has the shape of a disk with a diameter of 40 mm and a total surface area of 2513 mm$^2$ before the immersion in the volatile hydrophobic material. Absorption of volatile hydrophobic material for Examples 1 to 10 was performed at 55°C, and Examples 11-12 at 25°C. The total immersion time was 6 hours.

Table 1

| Raw Materials (wt.%) | Example | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| Castor oil | 80 | 80 | 80 | 75 | | | | | 39.3 | 78 | 39.3 | 78 |
| Epoxidized soybean oil | | | | | 90.4 | 82.6 | 82.6 | 82.6 | | | | |
| Citric acid | | | | | 4.8 | 8.7 | 8.7 | 8.7 | | | | |
| DESMODUR® eco N 7300[1] | 20 | 20 | 20 | 25 | | | | | | 22 | | 22 |
| Maleic anhydride | | | | | | | | | 12.4 | | 12.4 | |
| Polyethylene glycol diglycidyl ether (Mn 500) | | | | | | | | | 47.3 | | 47.3 | |
| Tetra-n-butylammonium bromide | | | | | | | | | 1 | | 1 | |
| Water (solvent)* | | | | | 4.8 | 8.7 | 8.7 | 8.7 | | | | |
| Volatile hydrophobic material | PPO | SPO | CNO | SPO | SPO | PPO | SPO | CNO | A | A | A | A |
| % loading by weight | 70.8 | 73 | 73.4 | 63.5 | 57.3 | 52.4 | 53.7 | 56.2 | 47.2 | 53.5 | 26.4 | 31.4 |

[1]DESMODUR® eco N 7300 is a bio-based polyisocyanate based on pentamethylene diisocyanate.
* The water is removed during the synthesis process

To confirm that the products obtained by the above methods are able to release volatile hydrophobic material, they were tested according to General Method E. Results are shown in Table 2.

Table 2

| | Example | | | |
|---|---|---|---|---|
| | **2** | **4** | **5** | **7** |
| Volatile Hydrophobic material release over 4 hours (mg/h/g) | 6.7 | 5.7 | 9.8 | 8.7 |

The degree of crosslinking of the solid articles may be influenced by adjusting the amount of crosslinking agent. As demonstrated in Tables 1 and 2, a higher degree of cross-linking results in a slower absorption/release of the volatile hydrophobic material. Examples 2 and 4 show polyurethane solid articles with lower (Example 2) and higher (Example 4) degrees of cross-linking, arising from the different amounts of DESMODUR® eco N 7300. Examples 5 and 7 show polyurethane solid articles with lower (Example 5) and higher (Example 7) degrees of cross-linking, arising from the different ratios of epoxidized soybean oil to citric acid. In both cases, lower absorption and release rates are observed for the solid articles having higher degrees of cross-linking. Therefore, solid articles having a lower degree of crosslinking may be used when a high release rate is desired. Similarly, solid articles having a higher degree of crosslinking may be used when sustained release over a long period is desired, though such products will require a longer absorption step during manufacture.

The immersion temperature influences the loading of the solid article as is demonstrated in Examples 9 to 12. Higher temperatures are believed to cause expansion of the solid article, facilitating absorption of volatile hydrophobic material within the polymer matrix. The higher the temperature during the absorption, the higher the loading percentage achieved for a given time. Examples 9 and 10 involved immersion at 55°C, while Examples 11 and 12 involved immersion at 25°C.

EXAMPLES 13-19

The solid articles used in Examples 13 to 19 (Table 3) were prepared according to General Method B. They were loaded with volatile hydrophobic material according to General Method D (vacuum method). In Examples 13 to 16, the solid article has the shape of a hemisphere with a diameter of 5 mm and a total surface area of $50.9\,mm^2$ before loading the volatile hydrophobic material. In Examples 17 to 19, the solid article has the shape of a disk with a diameter of 40 mm and a total surface area of $2513\,mm^2$ prior hydrophobic material loading. In Examples 13 to 17, the solid article was pre-heated at 100°C for 5 min. Examples 18 and 19 had no pre-treatment. In Examples 13 to 16, solid articles and volatile hydrophobic material were introduced in a vacuum bag (shrinkable bag, ref. 172030, Bolsemack, Spain) at a weight ratio of 40:60 solid article : volatile hydrophobic material. In Examples 17 to 19, the weight ratio was of 80:20.

Table 3

| Raw Materials (wt. %) | Example No. | | | | | | |
|---|---|---|---|---|---|---|---|
| | **13** | **14** | **15** | **16** | **17** | **18** | **19** |
| Castor oil | | | | 75 | 78 | 78 | 39.3 |
| Epoxidized soybean oil | 82.6 | 82.6 | 82.6 | | | | |
| Citric acid | 8.7 | 8.7 | 8.7 | | | | |
| DESMODUR® eco N 7300[1] | | | | 25 | 22 | 22 | |
| Maleic anhydride | | | | | | | 12.4 |
| Polyethylene glycol diglycidyl ether (Mn 500) | | | | | | | 47.3 |
| Tetra-n-butylammonium bromide | | | | | | | 1 |
| Water (solvent)* | 8.7 | 8.7 | 8.7 | | | | |
| Volatile hydrophobic material | PPO | SPO | CNO | SPO | A | A | B |
| % wt loading | 41.2 | 50.4 | 55.0 | 56.7 | 19.5 | 17 | 17.3 |

[1]DESMODUR® eco N 7300 is a bio-based polyisocyanate based on pentamethylene diisocyanate.
* The water is removed during the synthesis process

As evidenced by comparing Examples 17 and 18, pretreatment at elevated temperature (Example 17) results in higher volatile hydrophobic material loading for an equal time. This is believed to be due to expansion of the polymer gel matrix facilitating absorption.

To confirm that the products obtained by the above methods are able to release volatile hydrophobic material, they were tested according to General Method E. Results are shown in Table 4.

Table 4

| | Example No. | | | | | | | |
|---|---|---|---|---|---|---|---|---|
| | **4** | **6** | **7** | **8** | **13** | **14** | **15** | **16** |
| Hydrophobic material release over 20 hours (mg/h/g) | 3.7 | 2.8 | 4.6 | 2.3 | 2.07 | 2.7 | 1.09 | 1.8 |

Examples 4, 6, 7 and 8 prepared by the immersion method provide faster release of volatile hydrophobic material as compared to Examples 13 to 16 prepared by vacuum absorption. Thus, the immersion method may be particularly suited to applications where a faster release rate is desirable, such as air freshening products or other home applications.

The vacuum method may be particularly suited to applications where a slower release rate, and thus a higher lifetime, is desirable, such as pest repellant products (e.g. pheromone release in crop fields). Without being bound by theory, the vacuum method may cause the volatile hydrophobic material to be absorbed more deeply into the center of the polymer gel matrix, causing a slower release.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

**Claims**

1.  A method of preparing a product for the release of volatile hydrophobic material, comprising the steps:

    i) providing a solid article comprising a polymer gel matrix formed from a chemically cross-linked material; and
    ii) absorbing the volatile hydrophobic material into the solid article by contacting the solid article with liquid phase volatile hydrophobic material until the solid article comprises at least 5 wt. % of the volatile hydrophobic material, based on a final weight of the solid article,

    wherein:

    the solid article is non-porous; and
    the product is able to provide release of the volatile hydrophobic material by volatilization of the volatile hydrophobic material from the solid article at a temperature of 25°C, a relative humidity of 60%, and atmospheric pressure.

2.  The method according to claim 1, wherein contacting the solid article with liquid phase volatile hydrophobic material is conducted until the solid article comprises at least 15 wt. % of the volatile hydrophobic material, based on a final weight of the solid article.

3.  The method according to claim 1 or 2, wherein absorbing the volatile hydrophobic material into the solid article comprises contacting at least 70% of a surface area of the solid article with the liquid phase volatile hydrophobic material,
    optionally wherein absorbing the volatile hydrophobic material into the solid article comprises contacting at least 90% of a surface area of the solid article with the liquid phase volatile hydrophobic material.

4.  The method according to any one of the preceding claims, wherein the solid article is contacted with a liquid phase volatile hydrophobic material that comprises no more than 10 wt. % solvent,

    optionally no more than 5 wt. % solvent;
    more optionally no more than 3 wt. % solvent,
    most optionally no more than 1 wt. % solvent.

5.  The method according to any one of the preceding claims, wherein step ii) comprises immersing the solid article in the volatile hydrophobic material,
    optionally wherein the method further comprises the steps:

iiia) removing the solid article from the volatile hydrophobic material; and
iva) drying the solid articles to remove surface liquid volatile hydrophobic material.

6. The method according to claim 5, wherein step ii) is conducted at a temperature of from 10°C to 70°C,

   optionally at a temperature of from 15°C to 60°C,
   more optionally at a temperature of from 20°C to 55°C.

7. The method according to claim 5 or 6, wherein step ii) is conducted:

   until the solid article comprises at least 25 wt. % (e.g. at least 30 wt. %) of the volatile hydrophobic material, based on a final weight of the solid article; and/or
   for a time period of from 30 minutes to 24 hours.

8. The method according to any one of claims 1 to 4, wherein step ii) comprises allowing the volatile hydrophobic material to be absorbed by the solid article under a reduced pressure, optionally wherein the reduced pressure is a pressure of from $10^{-8}$ mbar ($10^{-9}$ kPa) to about 10 mbar (1 kPa).

9. The method according to claim 8, wherein step ii) comprises:
   iia) placing the solid article and volatile hydrophobic material into a vacuum bag;

   iib) applying the reduced pressure to the vacuum bag and sealing the vacuum bag;
   iic) allowing the volatile hydrophobic material to be absorbed into the solid article until the solid article comprises at least 5 wt. % of the volatile hydrophobic material, based on a final weight of the solid article.

10. The method according to claim 8 or 9, wherein the step of allowing the volatile hydrophobic material to be absorbed by the solid article under a reduced pressure is conducted at a temperature of from 30°C to 80°C,

    optionally from 40°C to 70°C,
    more optionally from 45°C to 60°C,
    such as about 50°C.

11. The method according to any one of claims 8 to 10, wherein the step of allowing the volatile hydrophobic material to be absorbed by the solid article under a reduced pressure is conducted:
    until the solid article comprises at least 20 wt. % (e.g. at least 25 wt. %) of the volatile hydrophobic material, based on a final weight of the solid article; and/or
    for a time period of from 60 minutes to 600 minutes.

12. The method according to any one of the previous claims, wherein the chemically cross-linked material is derived from a natural oil,
    optionally wherein the chemically cross-linked material is selected from the group consisting of a polyurethane, a polyester, and a polyester-polyether copolymeric material.

13. The method according to any one of the previous claims, further comprising a step of heating the solid article to a temperature of at least 70°C before contacting the solid article with the volatile hydrophobic material,
    optionally wherein the solid article is heated to a temperature of from 80°C to 120°C for a time period of from 3 minutes to 30 minutes.

14. A solid article obtainable by a method according to any one of the preceding claims, wherein the solid article comprises at least 5 wt. % of the volatile hydrophobic material, based on a final weight of the solid article.

15. Use of a solid article according to claim 14 for the release of a volatile hydrophobic material,
    optionally wherein the volatile hydrophobic material is selected from the group consisting of a perfume, an essential oil, an insect repellant and a pheromone.

**EUROPEAN SEARCH REPORT**

Application Number

**EP 24 38 2694**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | EP 0 904 771 B1 (SHOWA DENKO KK [JP])<br>18 June 2003 (2003-06-18)<br>* page 2, paragraph 1 *<br>* page 3, paragraph 11-13 *<br>* page 4, paragraph 18 - page 5, paragraph 26 *<br>* page 5, paragraph 29-32 *<br>* page 6, paragraph 37-40; examples 1-14; tables 1-2 *<br>* claims 1-7 * | 1-3,5-7,<br>13-15<br>9,10 | INV.<br>A61L9/04<br>A61L9/012<br>A61K8/04<br>A61Q13/00<br>C11D3/37<br>C11D3/50 |
| X<br><br>A | JP 2022 539602 A (BASF SE [DE])<br>12 September 2022 (2022-09-12)<br>* abstract; claims 1-14; examples production examples 1-3 * | 1-7,12,<br>13,15<br>9,10 | |
| X<br><br>A | JP 6 343847 B2 (KAO CORP)<br>20 June 2018 (2018-06-20)<br>* the whole document * | 1-8,11,<br>13-15<br>9,10 | |
| X | JP 2012 254184 A (ST CORP)<br>27 December 2012 (2012-12-27)<br>* abstract; claims 1-17; examples 1-5 * | 1,4,14,<br>15 | TECHNICAL FIELDS SEARCHED (IPC)<br><br>A61L |
| A | US 2022/016295 A1 (FERNANDEZ PRIETO SUSANA [BE] ET AL) 20 January 2022 (2022-01-20)<br>* the whole document * | 1-15 | A61Q<br>C11D<br>A61K |

The present search report has been drawn up for all claims

| Place of search | Date of completion of the search | Examiner |
|---|---|---|
| Munich | 12 December 2024 | Handrea-Haller, M |

## ANNEX TO THE EUROPEAN SEARCH REPORT
## ON EUROPEAN PATENT APPLICATION NO.

EP 24 38 2694

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2024

| Patent document cited in search report | | Publication date | Patent family member(s) | | Publication date |
|---|---|---|---|---|---|
| EP 0904771 | B1 | 18-06-2003 | AT | E243023 T1 | 15-07-2003 |
| | | | DE | 69815614 T2 | 06-05-2004 |
| | | | EP | 0904771 A1 | 31-03-1999 |
| | | | JP | H1171572 A | 16-03-1999 |
| | | | TW | 367250 B | 21-08-1999 |
| JP 2022539602 | A | 12-09-2022 | BR | 112021023788 A2 | 08-02-2022 |
| | | | CN | 114096234 A | 25-02-2022 |
| | | | EP | 3996660 A1 | 18-05-2022 |
| | | | JP | 2022539602 A | 12-09-2022 |
| | | | US | 2022288549 A1 | 15-09-2022 |
| | | | WO | 2021009040 A1 | 21-01-2021 |
| JP 6343847 | B2 | 20-06-2018 | JP | 6343847 B2 | 20-06-2018 |
| | | | JP | 2015120847 A | 02-07-2015 |
| JP 2012254184 | A | 27-12-2012 | JP | 6017120 B2 | 26-10-2016 |
| | | | JP | 2012254184 A | 27-12-2012 |
| US 2022016295 | A1 | 20-01-2022 | EP | 3431143 A1 | 23-01-2019 |
| | | | JP | 6899934 B2 | 07-07-2021 |
| | | | JP | 2020524680 A | 20-08-2020 |
| | | | KR | 20200018621 A | 19-02-2020 |
| | | | KR | 20210152017 A | 14-12-2021 |
| | | | US | 2019022265 A1 | 24-01-2019 |
| | | | US | 2022016295 A1 | 20-01-2022 |
| | | | WO | 2019016707 A1 | 24-01-2019 |

EPO FORM P0459

For more details about this annex : see Official Journal of the European Patent Office, No. 12/82

**EP 4 670 746 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11173224 B2

**Non-patent literature cited in the description**

- **HAMMOUDA, B.** Insight into Clustering in Poly(ethylene oxide) Solutions. *Macromolecules*, 2004, vol. 37, 6932-6937